# EUROPEAN PATENT APPLICATION

(11) **EP 4 046 489 A1**
(43) Date of publication of application: **24.08.2022**
(21) Application number: 21194867.4
(22) Date of filing: 03.09.2021
(51) Int. Cl.: A01N 33/12, A01N 55/00, A01N 59/00, A61K 31/14, A61K 31/695, A61K 33/40, A61L 2/18, A01N 31/14, A01P 1/00

(54) **SURFACE DISINFECTANT AND HAND SANITIZER COMBATTING CONTACT SPREAD**

(30) Priority: 19.02.2021 US 202117180161; 30.04.2021 WO PCT/IB2021/053643
(71) Applicant: Zoono Group Ltd., 2013 Bottany, South Auckland (NZ)
(72) Inventor: Hyslop, Paul, Auckland (NZ)
(74) Representative: J A Kemp LLP

(57) **Abstract**

The technical filed is a composition which has properties to act as a surface disinfectant and hand sanitizer for combatting contact infection spread. The surface disinfectant overcomes the problem of immediately killing a broad range of microbes on the surface and thereafter protecting that surface for 30 days. The hand sanitizer overcomes the problem of sanitizing hands against a broad range of microbes for 24 hours. The two can work together to safeguard a community from contact infection spread.

## Description

### TECHNICAL FIELD

This invention pertains generally to protecting a community from the contact spread of infection and more particularly to kill and protect surface disinfectants and hand sanitizers.

### BACKGROUND ART

Human beings compete in this world with microbes which infect human beings causing illness and death. A route for infection spread is human beings contacting surfaces laden with an infectious microbe and contacting other human beings who are infected with a microbe. Beginning in the year 2020 and continuing at least up to the time of the filing of the application matriculating into this patent, the worldwide human population was plagued with a COVID-19 virus pandemic.

World Intellectual Property Organization publication WO2011060132 pertains to a disinfecting apparatus. Within this patent publication is the teaching of a disinfectant solution composed of ingredients selected from a long list of potential ingredients. This long list includes benzalkonium chloride, hydrogen peroxide and quaternary ammonium silane 3-(tri-methoxysilyl) propyldimethyl octadecyl ammonium chloride. There is no guidance on which ingredients to select and no guidance on the percentages of the same in a composition.

US Patent 9,445,600 teaches a kill and protect disinfectant composition comprised of quaternary ammonium silane 3-(tri-methoxysilyl) propyldimethyl octadecyl ammonium chloride in a concentration between 0.1% to 2.0% with 0.5% preferred, hydrogen peroxide in a concentration between 0.5% to 7.5% with 4.0% preferred and other ingredients.

US 6,994,890 teaches a kill and protect disinfectant composition comprised of quaternary ammonium silane 3-(tri-methoxysilyl) propyldimethyl octadecyl ammonium chloride in a concentration of 1.0% and hydrogen peroxide in a concentration between 3.00% to 6.0%.

US Patent 8,999,357 teaches a kill and protect disinfectant comprised of quaternary ammonium silane 3-(tri-methoxysilyl) propyldimethyl octadecyl ammonium chloride in a concentration of 4.0%, benzalkonium chloride in a concentration of 6.0% and other ingredients.

US 8,383,164 teaches a kill disinfectant comprised of an aqueous solution of benzalkonium chloride in a concentration between 0.1% to 0.3, hydrogen peroxide in a concentration between 0.1% to 3.00%.

A deficiency in the art is the effectiveness of kill and protect surface disinfectants and hand sanitizers to combat the contact infection spread.

A deficiency in the art is the duration and longevity with effectiveness of kill and protect surface disinfectants and hand sanitizers to combat the contact infection spread.

A related deficiency in the art is a lack of sufficient effectiveness of kill and protect surface disinfectants and hand sanitizers to control, reduce, and ideally eliminate microbes such that government officials do not implement, lift and/or relax restrictions on individuals.

Accordingly, there exists a need for improved formulations of kill and protect surface disinfectants and hand sanitizers to reduce, and ideally eliminate, deleterious microbes from surfaces and hands.

There exists a need for an improved formulation of a kill and protect surface disinfectants having a 30-day longevity.

There exists a need for an improved formulation of a hand sanitizer having a 24-hour longevity.

There exists a need for a sufficiently effective kill and protect surface disinfectant and hand sanitizer to control, reduce, and ideally eliminate microbes such that government officials do not implement, lift and/or relax restrictions on individuals.

There exists a related need for a system, or combination, of a kill and protect surface disinfectant and a hand sanitizer, to disinfect surfaces and hands to combat contact infection spread.

The present invention satisfies these needs, as well as others, and generally overcomes the presently known deficiencies in the art.

### SUMMARRY OF THE INVENTION

The present invention is directed to kill and protect surface disinfectants and hand sanitizers. The invention is also directed to combined utilization for combatting contact infection spread.

Objectives of the present invention are a kill and protect surface disinfectant and a hand sanitizer with each having improved effectiveness.

An object of the present invention is an improved formulation of a kill and protect surface disinfectants having a 30-day longevity.

An object of the present invention is an improved formulation of a hand sanitizer having a 24-hour longevity.

An object of the present invention is a kill and protect surface disinfectant and a hand sanitizer which are utilizable in combination or as a system so as to combat contact infection spread.

An object of the present invention is kill and protect surface disinfectants and hand sanitizers to control, reduce, and ideally eliminate microbes such that government officials do not implement, lift and/or relax restrictions on individuals.

One aspect of the present invention is a kill and protect surface disinfectant composition. This kill and protect surface disinfectant composition is comprised of between about 0.20% (w/w) to about 1.00% (w/w) benzalkonium chloride; between about 0.25% (w/w) to about 3.00% (w/w) hydrogen peroxide; between about 0.25% (w/w) to about 0.75% (w/w) 3-(tri-methoxysilyl)propyldimethyl octadecyl ammonium chloride and between about 95.25% (w/w) to about 99.30% deionized water. A longevity of at least about 30 days is achievable.

Another aspect of the present invention is a hand sanitizer composition. This hand sanitizer composition is comprised of between about 0.05% (w/w) to about 0.20% (w/w) benzalkonium chloride; between about 0.20% (w/w) to about 0.50% (w/w) 3-(tri-methoxysilyl)propyldimethyl octadecyl ammonium chloride; between about 0.25% (w/w) to about 0.75% (w/w) phenoxyethanol and between about 98.55% (w/w) to about 99.5% (w/w) deionized water. Optionally, a fragrance can be added with a commensurate reduction of deionized water. A longevity of at least about 24 hours is achievable.

Another aspect of the present invention is a method of providing a community benefit towards controlling the spread of infection where the infection spreads by humans touching static surfaces, e.g., shelving, displays and merchandise in a store, the intra-personal touching of hands to orifices such as mouths, noses, ears and eye orbitals and the inter-personal touching of hands and other body parts e.g., hand shaking and hugging. The method is comprised of the steps of sanitizing surfaces with an embodiment of a kill and protect surface disinfectant composition according to the present invention and humans sanitizing their hands with a hand sanitizer composition according to an embodiment of this invention.

The previously described versions of the present invention have many advantages which include improved formulations of kill and protect surface disinfectants and hand sanitizers to reduce, disinfect and ideally eliminate, deleterious microbes from surfaces and hands. The improvement being in kill effectiveness and longevity. Accordingly, there is a safeguarding of individual humans who uncontrollably wipe or touch a mouth, a nose or other orifice so as to afford entry to a pathogen which is on a hand. The surface disinfectant and hand sanitizer can be synergistically utilized together for the safeguarding of a community from contact infection spread.

These and other features, aspects and advantages of the present invention will become better understood with reference to the following description and appended claims

### MODES FOR CARRYING OUT THE INVENTION

The present invention is described more fully in the following disclosure. In this disclosure, there is a discussion of embodiments of the invention and references to accompanying in column drawings and tables related to embodiments of the invention. These specific embodiments are provided so that this invention will be understood by those skilled in the art. This invention is not limited to the specific embodiments set forth herein below and in the drawings. The invention is embodied in many different forms and should be construed as such with reference to the appended claims.

Embodiments of the present invention are for a kill and protect surface disinfectant and for a hand sanitizer. They are utilizable in the setting of a community comprised of humans and surfaces. Humans interact with the surfaces by touching the surfaces, e.g., shelving, displays and merchandise in a store. There is human to human inter-personal touching of hands and other body parts e.g., during hand shaking and hugging. The interacting is followed by the uncontrollable wiping or touching of a mouth, a nose or other orifice so as to afford entry to a pathogen into a human body and hence a pathogen infection and spread. The surface disinfectant and hand sanitizer are utilizable singling or in combination for combatting contact infection and spread. Protection is afforded to individuals. In addition, an overarching embodiment of this invention is a method of providing a community benefit towards controlling the spread of infection.

In the discussion that follows, "(w/w)" stands for the percent by weight of an ingredient in a composition and is based on the weight of the ingredient divided by the total weight of all ingredients in a composition with the dividend multiplied by 100. A percent by weight (w/w) range is inclusive of the endpoints and includes all points therebetween. IUPAC is an international federation that develops and maintains a naming system and nomenclature for molecules using terminology based upon constituent components. IUPAC naming is general used and has meaning to persons in the art. A CAS Number is a unique numerical identifier assigned by the Chemical Abstracts Service (CAS) to every chemical substance described in the open scientific literature. CAS numbers are general used and have meaning to persons in the art.

In general terms and for an overview, embodiments of the kill and protect surface disinfectant composition are comprised of the following major components: benzalkonium chloride, hydrogen peroxide, 3 Tri-Methoxysilyl)propyldimethyl octadecyl ammonium chloride, and deionized water. In the discussion that follows, each of these major components is discussed, along with other components/structures in the embodiments of this invention. Thereafter, there is a discussion on how to use the kill and protect surface disinfectant composition.

Benzalkonium chloride is one of two immediate kill agents in the kill and protect surface disinfectant composition. The other being hydrogen peroxide which is discussed herein below. Benzalkonium chloride (BZK) is a quaternary ammonium compound. The molecule has the chemical structure shown below: 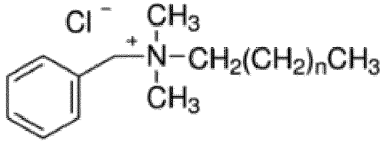 where n = 8, 10, 12, 16 and 18. The molecule has IUPAC names of N-Alkyl-N-benzyl-N,N-dimethylammonium chloride and Alkyldimethylbenzylammonium chloride.

Preferably, the benzalkonium chloride is that of the same which meets the description of Chemical Abstracts Service No. 68424-85-1. That is, quaternary ammonium compounds, benzyl-C12-16-alkyldimethyl, chlorides. As of the filing of the application for this patent, such benzalkonium chlorides are listed substances with the United States Environmental Protection Agency (EPA) and assigned tracking number 429654. Therefore, compounding with the same bodes well for getting regulatory approval and registration of a kill and protect surface disinfectant composition. More preferably, the alkyl component is C12 (dodecyl), C14 (Myristyl), a blend of 40% C12, 50% C14 and 10% C10.

As introduced above, benzalkonium chloride is a fast-acting microbicidal agent having have both bactericidal and virucidal activity, as well as being active against fungi and protozoa. A postulated mode of action is that benzalkonium chloride is a cationic surfactant that dissociates a microbial cellular membrane and thereby compromising cellular permeability controls and inducing leakage of cellular contents. Also postulated is that benzalkonium chloride disrupts/dissociates intermolecular interactions and denatures the tertiary structure of proteins. The result being the deactivation of enzymes essential to microbe respiratory and metabolic activities.

The concentration of the benzalkonium chloride in the kill and protect surface disinfectant composition ranges from about 0.20% percent by weight (w/w) to about 1.00% percent by weight (w/w). In a more preferred embodiment, the concentration of the benzalkonium chloride is about 0.50%. The benzalkonium chloride at the above concentrations in combination with the hydrogen peroxide (discussed below), and perhaps with other ingredients, at the concentrations and relative concentrations as taught herein below, is believed to act in a synergistic manner with disinfection that is heightened from that which can be expected based on the prior art.

Hydrogen peroxide is one of two immediate kill agents in the kill and protect surface disinfectant composition. The other being benzalkonium chloride which is discussed herein above. Hydrogen peroxide is a molecule with an oxygen-oxygen single bond and a single hydrogen bounded to each of the oxygens. Hydrogen peroxide has the chemical formula H₂O₂ or H2O2 and the chemical structure shown below: 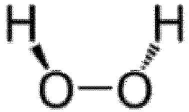 Hydrogen peroxide has a molecular weight in grams per mole of 34.01. Other names for hydrogen peroxide are perhydroxic acid and dihydrogen dioxide.

Preferably, the hydrogen peroxide is that of the same which meets the description of Chemical Abstracts Service No.7722-84-1. As of the filing of the application for this patent, such hydrogen peroxide is a listed substance with the United States Environmental Protection Agency (EPA)and assigned tracking number 153015. Therefore, compounding with the same bodes well for getting regulatory approval and registration of a kill and protect surface disinfectant composition.

Hydrogen peroxide is a strong disinfectant which possesses bactericidal, virucidal, sporicidal and fungicidal activity. This toxicity is attributed to hydrogen peroxide being an oxidizing agent under acidic conditions, including physiological acidic conditions. In particular, the hydrogen peroxide oxidizes in a microbe its proteins, membrane lipids, deoxynucleic acids (DNA) and ribonucleic acids rendering them nonfunctional.

The concentration of hydrogen peroxide in the kill and protect surface disinfectant composition ranges from about 0.25% percent by weight (w/w) to about 3.00% percent by weight (w/w). In a more preferred embodiment, the concentration of the hydrogen peroxide is about 0.50%. The combination of hydrogen peroxide in the foregoing concentrations and relative concentrations with respect to the benzalkonium chloride in the above taught concentrations and relative concentrations is believed to act in a synergistic manner with disinfection that is heightened from that which can be expected based on the prior art. Further, the combination of hydrogen peroxide in the foregoing concentrations and relative concentrations with respect to the concentrations and relative concentrations of 3-(tri-methoxysilyl)propyldimethyl octadecyl ammonium (discussed below), and perhaps other ingredients, is believed to act in a synergistic manner to enhance condensation/polymerization and binding of a formed micro-coating (discussed below) that is heightened from that which can be expected based on the prior art.

The quaternary ammonium silane 3-(tri-methoxysilyl)propyldimethyl octadecyl ammonium chloride is the long lasting protect agent of the kill and protect surface disinfectant composition. The molecule has the chemical structure shown below: 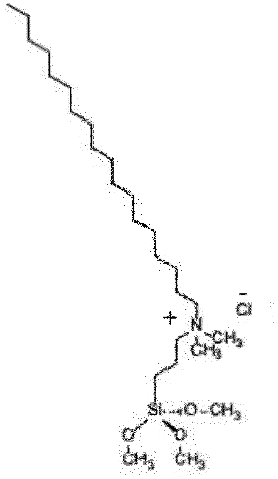 The molecule has the IUPAC name of 1-Octadecanaminium, N,N-dimethyl-N-[3-(trimethoxysilyl)propyl]-, chloride (1:1). The molecular formula is C26H58NO3Si.Cl and the molecular weight is 496.29.

Preferably, the quaternary ammonium silane 3-(trimethoxysilyl)propyldimethyl octadecyl ammonium chloride is that of the same which meets the description of Chemical Abstracts Service No. 27668-52-6. As of the filing of the application for this patent, such quaternary ammonium silane 3-(trimethoxysilyl)propyldimethyl octadecyl ammonium chloride is a listed substances with the United States Environmental Protection Agency (EPA) and assigned tracking number 246561. Therefore, compounding with the same bodes well for getting regulatory approval and registration of a kill and protect surface disinfectant composition.

The believed mode of action of the 3-(tri-methoxysilyl)propyldimethyl octadecyl ammonium chloride is as follows. The following discourse on the believed mode of action necessarily overlaps with a discourse on method of use the kill and protect surface disinfectant composition and there is a further discourse on method of use below. The 3-(tri-methoxysilyl)propyldimethyl octadecyl ammonium chloride and deionized water (discussed below) components does not require that there be mixing of components; i.e., the composition is ready to use. The composition is applied to an inanimate surface. The applying is done by misting, spraying, brushing or dipping. When the composition dries, a condensation reaction occurs where there is the formation of a micro thin coating of polymerized monomers of 3-(trimethoxysilyl)propyldimethyl octadecyl ammonium with strong covalent bonds between the monomers. The octadecyl alkyl side chain lineup pointing away from the surface. This can be likened to having millions of sword-shaped "road spikes" on the surface. At the base of each of these "swords" is a positively charged quaternary carbon unit.

Each of positively charged quaternary carbon units performs a dual function. One function is to attach the micro-coating to commonly found surfaces which bear a negative charge. The other is to attract/trap microbes with a negatively charged outer membrane. Typically, microbes have an outer membrane comprised of lipoproteins which have long chain fatty acids and glycerides that are anionic in nature.

The alkyl side chain then pierces the outer membrane of the attracted/trapped microbe with organism lysis. The microbe is killed via a form of electrostatic electrocution. The same monomer in the polymerized micro-coating is capable of attacking a new microbe again and again with only a single application. Because it does not kill the organisms by poisoning with toxic chemicals, they cannot adapt to it so there is no possibility of mutation and consequently no possibility of superbugs forming.

In more detail, the 3-(tri-methoxysilyl)propyldimethyl octadecyl ammonium chloride first undergoes an hydrolysis in conjunction with the deionized water (discussed below) to enter into an equilibrium with 3-(tri-hydoxysilyl)propyldimethyl octadecyl ammonium chloride. This hydrolysis is depicted in the chemical drawing and chemical equation shown below: 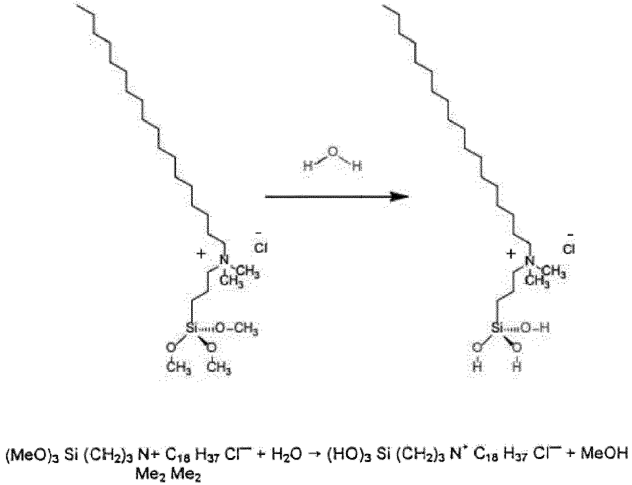 The condensation/polymerization reaction is depicted in the chemical drawing and chemical equation shown below:

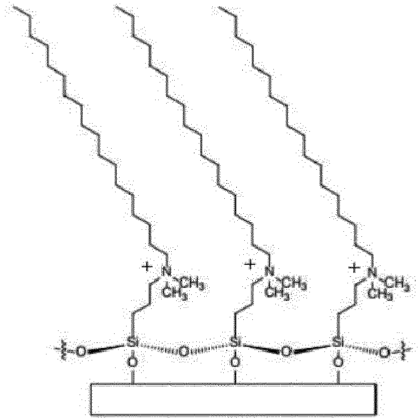

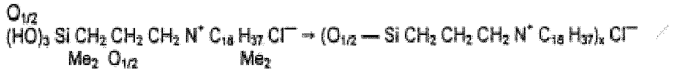

The concentration of 3-(tri-methoxysilyl)propyldimethyl octadecyl ammonium in the kill and protect surface disinfectant composition ranges from about 0.25% percent by weight (w/w) to about 0.75% percent by weight (w/w). In a more preferred embodiment, the concentration of the 3-(tri-methoxysilyl)propyldimethyl octadecyl ammonium is about 0.50%. The 3-(tri-methoxysilyl)propyldimethyl octadecyl ammonium in combination in the foregoing concentrations and relative concentrations with respect to the hydrogen peroxide in the above taught concentrations and relative concentrations is believed to act in a synergistic manner to enhance the bonding of the micro-coating onto surfaces that is heightened from that which can be expected based on the prior art. Also believed enhanced by the concentrations and relative concentrations of 3-(tri-methoxysilyl)propyldimethyl octadecyl ammonium and hydrogen peroxide as taught herein from that which can be expected based on the prior art is the durability of the micro-coating on various surfaces.

Deionized water is the base/solvent. Water is a molecule with an oxygen atom and two hydrogen atoms singling bounded to the oxygen atom. Water has the chemical formula H₂O or H2O. The molecule has the chemical structure shown below: 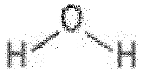 Water has a molecular weight in grams per mole of 18.01528. Being deionized, as a to-be-added ingredient to the composition, the concentration of the water approaches 100%. Accordingly, the melting point of deionized water approaches 0 °C (degrees Celsius) and the boiling point approaches 100 °C. At 20 °C, deionized water has a vapor pressure of 17.5 Torr. Preferably, the deionized water is that of the same which meets the description of Chemical Abstracts Service No. 7732-18-5.

As introduced above, the deionized water is a base/solvent into which the other ingredients are dissolved. The deionized water is also reactant in the hydrolysis discussed above 3-(tri-methoxysilyl)propyldimethyl octadecyl ammonium. The evaporation of the deionized water is integral with the condensation/polymerization of the 3-(tri-methoxysilyl)propyldimethyl octadecyl ammonium into a microcoating, as discussed above.

The concentration of deionized water in the kill and protect surface disinfectant composition ranges from about 95.25% percent by weight (w/w) to about 99.30% percent by weight (w/w). The particular concentration of deionized water is a function of the concentration of all other ingredients so that total of every ingredient, including deionized water, is 100%. Accordingly, concentrations are set for 3-(trimethoxysilyl)propyldimethyl octadecyl ammonium chloride between about 0.25% (w/w) to about 0.75% (w/w), benzalkonium chloride between about 0.25% (w/w) to about 0.75% (w/w) and hydrogen peroxide between about 0.25% (w/w) to about 0.75% (w/w) with deionized water being present in a concentration to achieve the concentrations set for the other ingredients. In a more preferred embodiment, where the concentration of 3-(tri-methoxysilyl)propyldimethyl octadecyl ammonium chloride is about 0.50% (w/w), the concentration all benzalkonium chloride is about 0.50% (w/w) and the concentration of hydrogen peroxide hydrogen peroxide about 0.50% (w/w), the concentration of deionized water is about 98.5% (w/w).

The kill and protect surface disinfectant composition (and hand sanitizers which our further discussed below) are manufactured by mixing and blending using standard equipment for chemical mixing and blending. That is, the compositions are made using for examples, liquid storage containers, ingredient and product handlers, in-line mixers, co-rotating twin-screw mixers, homogenizers, bottling operations, automation, electronic control and product storage, in the manner in accordance with that prescribed by the manufacturer(s) of the equipment. Preferably, the manufacturing facility is certified by the environmental protection agency or other regulatory agency having jurisdiction.

The typical method for using the kill and protect surface disinfectant composition is to apply the composition to a surface by misting, spraying, brushing or dipping (dipping is usually done with textiles.) The kill component of the composition, as discussed above, will kill microbes in a period of about 1 minute to about two hours, depending upon the nature of the surface and temperature.

The protect component of the composition, as discussed above, will react with oxides and hydroxyls on the surface of the surface through the above-described condensation reaction. The bonds formed are covalent and are resistant to rehydration. The protect component of the composition is generally capable of forming coatings on siliceous materials, e.g., glass and sand; metals; textile fibers made from cotton, cellulose, acetate, polyester, nylon, wool, rayon and acrylon; plastic surfaces, e.g. nylon, polyvinyl chloride, fiberglass, epoxy, polyester and rayon. On evaporation of the water solvent, a durable bonded coating is produced which last at least about 30 days.

The kill and protect surface disinfectant composition is believed, as a general proposition, but not necessarily to every listed species, to be active against:
(1) Gram Positive Bacteria, e.g., cacillus sp. (vegetative cell), corynebacterium diptheriae, micrococcus lutea, micrococcus sp., mycobacterium tuberculosis, mycobacterium smegmatis, propionibacterium acnes, staphylococcus aureus, staphylococcus epidermidis, streptococcus faecalis, streptococcus mutans, streptococcus pneumonia, streptococcus pyogenes;
(2) Gram Negative Bacteria, e.g., acinetobacter calcoaceticus, aeromonas hydrophilia, citrobacter deversus, citrobacter freundi, enterobacter aerogenes, enterobacter aglomerans, enterobacter cloacae, enterococcus, escherichia coli, klebsiella oxytoca, klebsiella pneumoniae, klebsiella terriena, legionella pneumophila, morganella morganii, proteus mirabilis, proteus vulgaris, psseudomonas aeruginosa, pseudomonas fluorscens, salmonella cholera suis, salmonella typhi, salmonella typhimurium, serratia liquifaciens, serratia marcescens, xanthomonas campestris;
(3) Viruses. e.g., adenovirus Type II & IV, bovine adenovirus Type I & IV, corona virus, COVID-19 (SARS-Cov-2), feline pneumonitis, herpes simplex Type I, herpes simplex Type II, human immunodeficiency virus HIV-1 (AIDS), influenza A2 (Aichi), influenza A2 (Asian), influenza B, mumps, parinfluenza (Sendai), rous S arcoma, reovirus Type I, SARS-Cov-2 (COVID-19), simian virus 40, vaccinia, MS2, PRD1, H1N1 (Swine Flu), norovirus, Middle East Respiratory Syndrome (MERS);
(4) Fungi, Algae, Mould, Yeast and Spores, e.g., alterania alternate, aphanizomenon s p., aspergillus flavus, aspergillus niger, aspergillus sydowi, aspergillus terreus, aspergillus versicolor, aspergillus verrucaria, aureobasidium pullans, candida albicans, candida pseudotropocalis, chaetomium globsum, cladosporium cladosporioides, chlorella vulgaris, dreschslera australiensis epidermophyton s p., gliomastix cerealis, gloeophyllum trabeum microsporum s p., microsporum audouinii, monilia grisea, Oscillatoria, penicillium chrysogenum, pencillium commune, penicillium funiculosum, penicillium pinophilium, penicillium variable, phoma fimeti, pithomyces chartarum, poria placenta, Scenedesmus, saccharonyces cerevisiae, scolecobasidium humicola Selenastrum s p., trichoderma viride, trichophyton interdigitale Trichophyton maidson, trichophyton mentogrophytes, trichophyton sp. 9 and
(5) Protozoa Parasites, e.g., cryptosporidium parvum (oocysts).

In general terms and for an overview, embodiments of the hand sanitizer composition are comprised of the following major components: benzalkonium chloride, quaternary silane 3-methoxysilyl)propyldimethyl octadecyl ammonium chloride, phenoxyethanol and deionized water. In the discussion that follows, each of these major components is discussed, along with other components/structures in the embodiments of this invention. Thereafter, there is a discussion on how to use the hand sanitizer.

The benzalkonium chloride is that as described above in connection with teaching the kill and protect surface disinfectant composition. The concentration of the benzalkonium chloride in the hand sanitizer composition ranges from about 0.05% percent by weight (w/w) to about 0.20% percent by weight (w/w). In a more preferred embodiment, the concentration of the benzalkonium chloride is about 0.10%. The benzalkonium chloride at the above concentrations in combination with the quaternary ammonium silane 3-methoxysilyl)propyldimethyl octadecyl ammonium chloride and/or other ingredients at the concentrations and relative concentrations as taught herein below is believed to act in a synergistic manner with a disinfection that is heightened from that which can be expected based on the prior art.

The quaternary ammonium silane 3-(tri-methoxysilyl)propyldimethyl octadecyl ammonium chloride is that as described above in connection with teaching the kill and protect surface disinfectant composition. The 3-(trimethoxysilyl)propyldimethyl octadecyl ammonium chloride acts as a liquid phase agent and does not condense or polymerize. It functions to kill in a similar manner to that when it is condensed or polymerized into a micro-coating.

The concentration of the 3-(tri-methoxysilyl)propyldimethyl octadecyl ammonium chloride in the hand sanitizer composition ranges from about 0.20% percent by weight (w/w) to about 0.50% percent by weight (w/w). In a more preferred embodiment, the concentration of 3-(tri-methoxysilyl)propyldimethyl octadecyl ammonium chloride is about 0.20%. The 3-(tri-methoxysilyl)propyldimethyl octadecyl ammonium chloride at the above concentrations in combination with the benzalkonium chloride and/or other ingredients at the concentrations and relative concentrations as taught herein is believed to act in a synergistic manner with disinfection that is heightened from that which can be expected based on the prior art.

The phenoxyethanol is a microbicidal agent/preservative, emulsifier and fixative for a fragrance. The chemical formula is C₈H₁₀O2 or C8H10O2. The molecule has the chemical structure shown below: 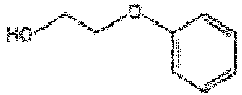 The molecular weight in grams per mole is 138.166. The molecule has the IUPAC name of 2-Phenoxyethanol. Preferably, the phenoxyethanol is that of the same which meets the description of Chemical Abstracts Service No. 122-99-6.

The phenoxyethanol is believed to perform the function of a microbicidal agent/preservative and active against Gram negative bacteria, Gram positive bacteria and yeast. The phenoxyethanol is also believed to be an emulsifier effective against local phases forming within the composition and keeping all ingredients in solution. The phenoxyethanol is also believed to have activity as a fixative for a fragrance.

The concentration of phenoxyethanol in the hand sanitizer composition ranges from about 0.25% percent by weight (w/w) to about 0.75% percent by weight (w/w). In a more preferred embodiment, the concentration of phenoxyethanol is about 0.50%. The phenoxyethanol at the above concentrations in combination with the benzalkonium chloride, 3-(tri-methoxysilyl)propyldimethyl octadecyl ammonium chloride and/or other ingredients at the concentrations and relative concentrations as taught herein is believed to act in a synergistic manner with disinfection that is heightened from that which can be expected based on the prior art.

The deionized water is that as described above in connection with teaching the kill and protect surface disinfectant composition and likewise functions as a base or solvent. The concentration of deionized water in the hand sanitizer composition ranges from about 98.55% percent by weight (w/w) to about 99.50% percent by weight (w/w). The particular concentration of deionized water is a function of the concentration of all other ingredients so that total of every ingredient, including deionized water, is 100%. Accordingly, targeted concentrations are set for benzalkonium chloride, 3-(tri-methoxysilyl)propyldimethyl octadecyl ammonium chloride and phenoxyethanol and deionized water being present in a concentration to achieve the concentrations set for the other ingredients. In a more preferred embodiment, where the benzalkonium chloride, 3-(tri-methoxysilyl)propyldimethyl octadecyl ammonium chloride and phenoxyethanol are at the above preferred concentrations and the preferred concentration of deionized water is therefore about 99.2% (w/w).

Optionally, and preferably, the hand sanitizer composition includes a fragrance. Examples of suitable fragrances are bergamot, gardenia, honeysuckle, jasmine, lavender, pear, peony, rosemary, sandalwood and vanilla. The concentration of fragrance in the hand sanitizer composition ranges from about 0.05% percent by weight (w/w) to about 0.15% percent by weight (w/w). In a more preferred embodiment, the concentration of fragrance is about 0.10%. The concentration of deionized water being proportionally less to achieve the foregoing targeted concentrations of fragrance.

The hand sanitizer composition is manufactured as discussed above for the manufacture of the kill and protect surface disinfectant.

The typical method for using the hand sanitizer is to apply generously to hands, rub the hands together so as to spread the hand sanitizer thoroughly about the hands and allow the hand sanitizer to remain in a liquid state on the hands for at least about a minute with longer being better. The hand sanitizer composition kills microbes for at least about 24 hours. It has activity against microbes as listed above.

The previously described versions of the present invention have many advantages. Amongst the many advantages are improved formulations of a hand sanitizer and surface sanitizer that provide longevity of at least about 24 hours for the hand sanitizer and at least about 30 days for the surface sanitizer. Accordingly, there is a safeguarding of individual humans who uncontrollably wipe or touch a mouth, a nose or other orifice so as to afford entry to a pathogen which is on a hand. There is a safeguarding of an entire community.

The present invention further provides for a composition comprised of:
a. between about 0.05% (w/w) to about 1.00% (w/w) benzalkonium chloride;
b. between about 0.25% (w/w) to about 3.00% (w/w) of a component selected from hydrogen peroxide and/or phenoxyethanol;
c. between about 0.20% (w/w) to about 0.75% (w/w) of 3-(trimethoxysilyl)propyldimethyl octadecyl ammonium chloride;
d. up to about 0.15% (w/w) fragrance;
e. about 95.10% (w/w) to about 99.50% (w/w) deionized water, such that the weight percentage total of all the ingredients is 100%.

Further provided is the use of such a composition as a disinfecting agent.

The nature of the components of the composition, the amounts of the components in the composition and the methods of using the composition areas described above in relation to the kill and protect surface disinfectant and hand sanitizer.

In one embodiment, the present invention provides a kill and protect surface disinfectant composition which is comprised of between about 0.05% (w/w) to about 1.00% (w/w) benzalkonium chloride; between about 0.25% (w/w) to about 3.00% (w/w) hydrogen peroxide; between about 0.25% (w/w) to about 0.75% (w/w) 3-(tri-methoxysilyl)propyldimethyl octadecyl ammonium chloride and between about 95.25% (w/w) to about 99.45% (w/w) deionized water, such that the weight percentage total of all the ingredients is 100%. A longevity of at least about 30 days is achievable.

In one embodiment, the kill and protect surface disinfectant comprises about 0.05% to about 0.20% (w/w) benzalkonium chloride and about 96.05% (w/w) to about 99.45% (w/w) deionized water.

In another embodiment, the kill and protect surface disinfectant composition comprises about 0.20% (w/w) to about 1.00% (w/w) benzalkonium chloride and about 95.25% (w/w) to about 99.30% (w/w) deionized water.

In one embodiment, the composition of the invention, the kill and protect surface disinfectant of the invention or the hand sanitizer of the invention comprises benzalkonium chloride, hydrogen peroxide and/or phenoxyethanol, 3-(trimethoxysilyl)propyldimethyl octadecyl ammonium chloride, and optionally fragrance in the amounts provided above and the balance of the composition is deionized water.

In a particular embodiment, the composition of the invention comprises:
benzalkonium chloride in an amount of about 0.05% to about 0.80% (w/w), preferably in an amount of 0.10% to 0.60% (w/w) and more preferably in an amount of 0.15% to 0.50% (w/w);
hydrogen peroxide and/ or phenoxyethanol in an amount of 0.25% to about 1.50% (w/w), preferably in an amount of 0.35% to 1.00% (w/w) and more preferably in an amount of 0.45% to 0.75% (w/w);
3-(tri-methoxysilyl)propyldimethyl octadecyl ammonium chloride in an amount of 0.20% to about 0.75% (w/w), preferably in an amount of 0.25% to 0.60% (w/w) and more preferably in an amount of 0.35% to 0.50% (w/w);
fragrance in an amount of up to 0.1% (w/w), preferably in an amount up to 0.05% (w/w),
and wherein the composition further comprises deionized water in an amount such that the weight percentage total of all the ingredients is 100%.

In a particular embodiment, the kill and protect surface disinfectant of the invention comprises:
benzalkonium chloride in an amount of about 0.05% to about 0.80% (w/w), preferably in an amount of 0.10% to 0.60% (w/w) and more preferably in an amount of 0.15% to 0.50% (w/w);
hydrogen peroxide in an amount of 0.25% to about 1.50% (w/w), preferably in an amount of 0.35% to 1.00% (w/w) and more preferably in an amount of 0.45% to 0.75% (w/w);
3-(tri-methoxysilyl)propyldimethyl octadecyl ammonium chloride in an amount of 0.20% to about 0.75% (w/w), preferably in an amount of 0.25% to 0.65% (w/w) and more preferably in an amount of 0.35% to 0.55% (w/w);
fragrance in an amount of up to 0.1% (w/w), preferably in an amount up to 0.05% (w/w),
and wherein the composition further comprises deionized water in an amount such that the weight percentage total of all the ingredients is 100%.

In a particular embodiment, the hand sanitizer of the invention comprises:
benzalkonium chloride in an amount of about 0.05% to about 0.20% (w/w), preferably in an amount of 0.05 to 0.15% (w/w) and more preferably in an amount of 0.10% to 0.15% (w/w);
phenoxyethanol in an amount of 0.25% to about 0.75% (w/w), preferably in an amount of 0.35% to 0.65% (w/w) and more preferably in an amount of 0.45% to 0.55% (w/w);
3-(tri-methoxysilyl)propyldimethyl octadecyl ammonium chloride in an amount of 0.20% to about 0.50% (w/w), preferably in an amount of 0.20% to 0.45% (w/w) and more preferably in an amount of 0.25% to 0.40% (w/w);
fragrance in an amount of up to 0.1% (w/w), preferably in an amount up to 0.05% (w/w),
and wherein the composition further comprises deionized water in an amount such that the weight percentage total of all the ingredients is 100%.

Additionally provided is the use of the kill and protect surface disinfectant as defined herein for surface sanitation.

Additionally provided is the use of the hand sanitizer as defined herein for hand sanitization.

### INDUSTRIAL APPLICABILITY

The following examples further describe and demonstrate embodiments within the scope of the present invention. The examples are given solely for the purpose of illustration and are not to be construed as limitations or restrictions of the present invention, as persons skilled in the art will quickly realize many variations thereof are possible that are all within the spirit and scope of the invention.

### Example 1

### (Biocidal Surface Bacterial)

Example 1 is a quantitative non-porous surface test for the evaluation of bactericidal activity of chemical disinfectants used in food, industrial, domestic and institutional areas. The testing protocol was standard method BS EN 13697. In more detail, BS EN 13697 is a surface test method for establishing whether a proposed disinfectant composition has or does not have bactericidal activity on non-porous surfaces. The BS EN 13697 laboratory test protocol closely simulates practical conditions of application. That is, chosen conditions (contact time, temperature, organisms etc.) reflect parameters which are found in practical situations including conditions which may influence the action of disinfectants.

An outline of the methodology of BS EN 13697 is as follows. A test suspension of bacteria in a solution of interfering substances is inoculated onto a test stainless steel surface and dried. A prepared sample of proposed disinfectant composition under test is applied in a manner which covers the dried film. The surface is maintained at the below specified temperature for the below defined period of time.

The surface is transferred to a previously validated neutralization medium so that the action of the disinfectant is immediately neutralized. The number of surviving organisms which can be recovered from the surface is determined quantitatively. The number of bacteria on a surface treated with hard water in place of the disinfectant is also determined and the reduction in viable counts attributed to the proposed disinfectant composition is the calculated difference.

The particular experiment conditions are presented in the below table:

| | |
|---|---|
| Disinfectant composition | A kill and protect surface disinfectant composition comprised of about 0.50% (w/w) benzalkonium chloride;about 0.50% (w/w) hydrogen peroxide; about 0.50% (w/w) 3-(trimethoxysilyl)propyldimethyl octadecyl ammonium chloride and about 98.5% (w/w) deionized water(percent by weight.) |
| Product diluent | Distilled Water |
| Test Concentrations | 100% (Neat,) 50%, 0.1% |
| Experimental Conditions | Clean |
| Interfering substances | Clean - Bovine Albumin 0.3 g/l (grams per litter) |
| Test Temperature | 20°C +/- 1°C (degrees Celsius) |
| Temperature of Incubation | Bacteria - 37°C +/- 1°C for 24hr to 48hrs (hours) |
| Identification of the reference strains | Pseudomonas aeruginosa ATCC 15442 |
| | Staphylococcus aureus ATCC 6538 |
| | Enterococcus hirae ATCC 10541 |
| | Escherichia coli ATCC 10536 |
| | Candida albicans ATCC 10231 |
| Contact times | Bacteria - 5 min ± 10s (minutes and seconds) |

The acceptance criteria for the disinfectant composition when tested according to BS EN 13697 is that the disinfectant composition shall demonstrate at least a 4 log₁₀ reduction in viable bacterial counts. The disinfectant composition has passed the test according to the acceptance criteria as outlined in the standard for bacterial isolates, when tested under clean conditions with a contact time of 5 minutes at a concentration of neat (100%.) The disinfectant composition has achieved a >3 log reduction against C.albicans, when tested under clean conditions with a contact time of 15 minutes at concentrations of neat and 50%.

### Example 2

### (Biocidal Surface Bacterial)

Example 2 is an evaluation of activity according to PN-EN 13727+A2:2015-12 (Poland.) The tested disinfectant composition was a kill and protect surface disinfectant composition comprised of about 0.50% (w/w) benzalkonium chloride; about 0.50% (w/w) hydrogen peroxide; about 0.50% (w/w) 3-(tri-methoxysilyl)propyldimethyl octadecyl ammonium chloride and about 98.5% (w/w) deionized water (w/w percent by weight,) where the benzalkonium chloride alkyl (C12-16) dimethylbenzyl ammonium chloride (ADBAC/BKC(C12-16).) The test methodology of this evaluation is dilution - neutralization. The neutralizer was Tween 80, 100 g/l; lecithin 30 g/l; L-histidine 1 g/l; sodium thiosulphate 5 g/1; phosphate buffer 10 ml/l (grams per liter and milliliters per liter.) The concentrations tested were 1% - 97% v/v (percent by volume.) The interfering substance was 0.3 g/l bovine albumin (grams per liter.) The test temperature was 20.0°C +/- 1.0°C (degrees Celsius.) The Contact time was 15 min +/-10 s (minutes and seconds.) The incubation method and parameters were a pour plate method at 37.0°C +/- 1.0°C for 48 h (degrees Celsius and hours.) The microbial strains in the test were Pseudomonas aeruginosa ATCC 15442, Staphylococcus aureus ATCC 6538, Enterococcus hirae ATCC 10541 and Escherichia coli K12 NCTC 10538.

The conclusion from the evaluation is that the tested disinfectant composition according to PN-EN 13727+A2:2015-12 at clean conditions (0.3 g/l bovine albumin), test temperature 20.0°C +/- 1,0°C, contact time 15 min, diluted in distilled water, is active (necessary reduction 5 log) against: Pseudomonas aeruginosa ATCC 15442 at 10% v/v and at 97% v/v; Staphylococcus aureus ATCC 6538 at 10% v/v and at 97% v/v; Enterococcus hirae ATCC 10541 at 10% v/v and at 97% v/v; Escherichia coli K12 NCTC 10538 at 10% v/v and at 97% v/v.

### Example 3

### (Biocidal Surface Viral)

Example 3 is a quantitative suspension test for evaluation of virucidal activity in the medical area. The testing protocol was standard method BS EN 14476. In more detail BS EN 14476 is a test method and the minimum requirements for virucidal activity of a chemical disinfectant composition that forms a homogenous physically stable preparation when diluted with hard water - or in the case of ready to use products that are not diluted when applied, - with water. Chemical disinfectant compositions can only be tested at a concentration of 80% (97% with a modified method for special cases) as some dilution is always produced by adding the test organisms and interfering substances. BS EN 14476 applies to areas and situations where disinfection is medically indicated; such as, hospitals, community medical facilities, dental institutions, school clinics, kindergartens, nursing homes, certain other workplace settings, certain home settings, certain laundries and certain kitchens.

An outline of the BS EN 14476 methodology is as follows. A sample of disinfectant composition is diluted in synthetic hard water in disinfectant composition diluted at point of use or water in the case of ready to use disinfectant composition is added to a test suspension of viruses in a solution of interfering substance. The mixture is maintained at one of the temperatures and contact times specified in the standard. At the end of this contact time, an aliquot is taken; the virucidal action in this portion is immediately suppressed by a validated method (dilutions of the sample in ice-cold cell maintenance medium). The dilutions are transferred into cell culture units either using monolayer or cell suspension. Infectivity tests are done either by plaque test or quantal tests. After incubation, the titres of infectivity are calculated according to Spearman and Kaber or by plaque counting. Reduction of virus infectivity is calculated from differences of lg (log) virus titres before (virus control) and after treatment with the product. The standard minimum spectrum of test organisms is Poliovirus, Adenovirus and Murine Norovirus.

The particular experiment conditions are presented in the below table:

| | |
|---|---|
| Disinfectant composition | A kill and protect surface disinfectant composition comprised of about 0.50% (w/w) benzalkonium chloride; about 0.50% (w/w) hydrogen peroxide; about 0.50% (w/w) 3-(trimethoxysilyl)propyldimethyl octadecyl ammonium chloride and about 98.5% (w/w) deionized water(percent by weight.) |
| Neutralization Method | Dilution |
| Product Diluent | Distilled water |
| Test Concentration | Neat (80%), Mid-range, Non active |
| Experimental Conditions | Clean |
| Interfering Substance | Clean 0.3g/l Bovine Albumin (grams per liter) |
| Test Temperature | 20°C +/- 1°C |
| Temperature of Incubation | 37°C +/- 1°C |
| Identification of the Viral Strains: | Modified vaccinia virus Ankara (MVA), ATCC VR-1508 |
| Contact Times | 5 minutes + 10s (seconds) |
| Stability and Appearance During Test | No Change Observed (Homogenous) |

The acceptance criteria for the disinfectant composition when tested according to BS EN 14476 is that the disinfectant composition when tested as above shall demonstrate at least a 4 log₁₀ reduction against the test virus. The test is deemed valid where all control requirements are met. The tested disinfectant composition has achieved a 4-log reduction against Vaccinia virus when tested under the condition stipulated above.

### Example 4

### (Biocidal and Longevity Surface Viral)

Example 4 is a study which evaluated the performance, characteristics, efficacy and usability to meet the needs of large numbers of people for a SARS-CoV-2 kill and protect surface disinfectant composition (sanitizer) comprised of about 0.50% (w/w) benzalkonium chloride; about 0.50% (w/w) hydrogen peroxide; about 0.50% (w/w) 3-(tri-methoxysilyl)propyldimethyl octadecyl ammonium chloride and about 98.5% (w/w) deionized water(percent by weight.) Three experiments were run (a positive control, a test and a negative control) where a total of 50 pairs of forceps were used. In the first experiment -a positive control-, twenty forceps were exposed to SARS COV 2 confirmed specimens and then swabbed and tested for SARS COV 2 at 1hr (hour) and 6 hours post exposure and for any DNA or RNA carrying microorganisms at day 5, 14 and 28 post exposure.

In the second experiment, twenty pairs of forceps were first treated using the disinfectant composition, let to dry and then contaminated with the same SARS COV 2 positive specimens. They were also swabbed and tested for SARS COV 2 at 1hr (hour) and 6 hours and for any DNA or RNA carrying microorganisms at day 5, 14 and 28 post exposure.

In a third experiment, as a negative control, 10 pairs of forceps were exposed to double distilled water, let to dry, swabbed and tested for SARS COV 2 at 1- and 6-hours post exposure and for any DNA or RNA carrying microorganisms at day 5, 14 and 28 post exposure.

All swabs were tested using the Abbott Realtime Polymerase Chain reaction for SARS COV 2. Two health care professionals were involved in the study and filled a questionnaire on usability. For the first experiment -a positive control-, the swabs taken from all the 20 pairs of forceps that were contaminated with SARS COV 2 specimens tested coronavirus positive at both the 1 and 6 hour intervals. They were also positive for DNA and RNA carrying microorganisms at day 5, 14 and 28 post exposure (explained as SARS COV 2 and/or contamination by microbes from a nonsterile environment.) In the second experiment, swabs taken from all the 20 pairs of forceps that were first shielded with the surface sanitizer and then contaminated with SARS COV 2 specimens tested coronavirus negative at both the 1 and 6 hour intervals. They were also negative for any DNA and RNA carrying microorganisms at day 5, 14 and 28 post exposure (explained as SARS COV 2 and contamination by microbes from a nonsterile environment being killed.) Swabs taken from the uncontaminated forceps -negative control- all tested negative at 1 and 6 hours. Each of the forceps was also found positive for DNA and RNA carrying microorganisms at day 5, 14 and 28 post exposure (explained as contamination from a nonsterile environment.)

In the foregoing, the efficacy of the tested kill and protect disinfectant composition for SARS-CoV-2 was 100% at 1 and 6 hours. The efficacy against any microorganism at days 5, 14 and 28 was also 100%.

### Example 5

### (Longevity Surface Bacterial)

Examples 5 is an analysis of longevity surface bacterial. The testing methodology was ISO 21149. A description of the sample tested is a kill and protect surface disinfectant composition (surface sanitizer and protectant) comprised of about 0.50% (w/w) benzalkonium chloride; about 0.50% (w/w) hydrogen peroxide; about 0.50% (w/w) 3-(tri-methoxysilyl)propyldimethyl octadecyl ammonium chloride and about 98.5% (w/w) deionized water (percent by weight.) The test results are as follows.

| Slide control (test organism only) | Tested kill and protect surface disinfectant composition and test organism | Log reduction between slide control recovery level and tested disinfectant composition recovery level (cfu/ml) | % reduction between slide control recovery level and tested disinfectant composition recovery level (cfu/ml) |
|---|---|---|---|
| Escherichia coli and contact time 30 days | | | |
| 1.3 x 10⁵ cfu/mL (log 5.1) | No recovery of Organism (log 1) | 5.1 | 100 |

| Pseudomonas aeruginosa and contact time 30 days | | | |
|---|---|---|---|
| 1.1 x 10⁵ cfu/mL (log 5.0) | No recovery of Organism (log 1) | 5.0 | 100 |

| Staphylococcus aureus and contact time 30 days | | | |
|---|---|---|---|
| 1.0 x 10⁵ cfu/mL (log 5.0) | No recovery of Organism (log 1) | 5.0 | 100 |

| Enterococcus faecalis and contact time 30 days | | | |
|---|---|---|---|
| 1.8 x 10⁵ cfu/mL (log 5.3) | No recovery of Organism (log 1) | 5.3 | 100 |

| Listeria monocytogenes and contact time 30 days | | | |
|---|---|---|---|
| 2.1x10⁵ cfu/mL (log 5.3) | No recovery of Organism (log 1) | 5.3 | 100 |

| | | | |
|---|---|---|---|
| (cfu/ml - colony forming units per milliliter) | | | |

### Example 6

### (Bacterial Biocidal and Longevity Activity Hand Sanitizer)

Example 6 is testing having the objective of determining the antibacterial efficacy of a hand sanitizer comprised of about 0.10% (w/w) benzalkonium chloride; about 0.50% (w/w) phenoxyethanol; about 0.20% (w/w) 3-(trimethoxysilyl)propyldimethyl octadecyl ammonium chloride and about 99.2% (w/w) deionized water. The testing methodology is in accordance with BSEN 1276. The test involved the preparation of a standard suspension of test organisms containing 1.5 - 5.0 x 10⁸ cells per ml (milliliter) of the four standard bacteria Escherichia coli K12 NCTC 10538, Enterococcus hirae ATCC 10541, Pseudomonas aeruginosa ATCC 15442, Staphylococcus aureus ATCC 6538 and Salmonella typhimurium ATCC 13311.

In overview, the test procedure was as follows. A sample of hand sanitizer as delivered or diluted to a test suspension of bacteria in a solution of an interfering substance. The mixture is maintained at 20 degrees Celsius for 24 hrs. At the end of this contact time an aliquot was taken and the bactericidal or bacteriostatic activity in this portion is immediately neutralized or suppressed by a validation method. After 5 mins (minutes) of neutralization time 1 mL (milliliter) of the sample mixture was taken and performed pour plate method. Further kept for incubation at 37°C (degrees Celsius) for 48 hrs (hours). The method of choice is dilution neutralization. The numbers of the surviving bacteria in the sample are determined and the reduction is calculated.

Particular experiment conditions are presented in the below table:

| | |
|---|---|
| Clean condition | 0.3 g/L (grams per liter): Bovine Serum Albumin |
| Agar | Tryptone Soya Agar |
| Neutralizers | Polysorbate 80, 30 g/l (grams per liter) + lecithin, 3 g/l + Sodium thioglycolate 30g/L prepared in de-ionized water and autoclaved for 15 minutes at 121°C (degrees Celsius) |

On the basis of the results it can be concluded that the tested specimen was observed with a 6 log reduction in bacterial count at 24 hrs (hours) contact time against Escherichia coli K12, Enterococcus hirae, Pseudomonas aeruginosa, Staphylococcus aureus, and Salmonella typhimurium.

### Example 7

### (Bacterial Biocidal Activity Hand Sanitizer)

Example 7 is an evaluation of activity according to PN-EN 13727+A2 of a hand sanitizer comprised of about 0.10% (w/w) benzalkonium chloride; about 0.50% (w/w) phenoxyethanol; about 0.20% (w/w) 3-(tri-methoxysilyl)propyldimethyl octadecyl ammonium chloride and about 99.2% (w/w) deionized water. The test method was dilution-neutralization. The hand sanitizer diluent in the test was distilled water. Concentrations tested were 1% - 97% v/v (percent by volume.) The interfering substance was 0.3 g/l (grams per liter) bovine albumin. The test temperature was 20.0°C +/- 1.0°C (degrees Celsius.) The contact time was 60 s +/- 5 s (seconds.) The incubation parameters were 37.0°C +/- 1.0°C (degrees Celsius,) 48 h (hours) and pour plate method. The microbial strains in the test were Pseudomonas aeruginosa ATCC 15442, Staphylococcus aureus ATCC 6538, Enterococcus hirae ATCC 10541 and Escherichia coli K12 NCTC 10538.

The conclusion from the test results is that the hand sanitizer according to PN-EN 13727+A2:2015-12 at clean conditions (0.3 g/l bovine albumin), test temperature 20.0°C ± 1.0°C, contact time 60 s, diluted in distilled water, is active (necessary reduction 5 log) against Pseudomonas aeruginosa ATCC 15442 at 97% v/v, Staphylococcus aureus ATCC 6538 at 97% v/v, Enterococcus hirae ATCC 10541 at 97% v/v and Escherichia coli K12 NCTC 10538 at 97% v/v.

### Example 8

### (Viral Biocidal Activity Hand Sanitizer)

Example 8 is a quantitative suspension test for evaluation of virucidal activity in the medical area. The testing protocol was standard method BS EN 14476. In more detail BS EN 14476 is a test method and the minimum requirements for virucidal activity of a chemical disinfectant composition that forms a homogenous physically stable preparation when diluted with hard water - or in the case of ready to use products that are not diluted when applied, - with water. Chemical disinfectant compositions can only be tested at a concentration of 80% (97% with a modified method for special cases) as some dilution is always produced by adding the test organisms and interfering substances. BS EN 14476 applies to areas and situations where disinfection is medically indicated; such as, hospitals, community medical facilities, dental institutions, school clinics, kindergartens, nursing homes, certain other workplace settings, certain home settings, certain laundries and certain kitchens.

An outline of the BS EN 14476 methodology is as follows. A sample of disinfectant composition is diluted in synthetic hard water in disinfectant composition diluted at point of use or water in the case of ready to use disinfectant composition is added to a test suspension of viruses in a solution of interfering substance. The mixture is maintained at one of the temperatures and contact times specified in the standard. At the end of this contact time, an aliquot is taken; the virucidal action in this portion is immediately suppressed by a validated method (dilutions of the sample in ice-cold cell maintenance medium). The dilutions are transferred into cell culture units either using monolayer or cell suspension. Infectivity tests are done either by plaque test or quantal tests. After incubation, the titres of infectivity are calculated according to Spearman and Kaber or by plaque counting. Reduction of virus infectivity is calculated from differences of lg (log) virus titres before (virus control) and after treatment with the product. The standard minimum spectrum of test organisms is Poliovirus, Adenovirus and Murine Norovirus.

The particular experiment conditions are presented in the below table:

| | |
|---|---|
| Disinfectant composition | A hand sanitizer comprised of about 0.10% (w/w) benzalkonium chloride; about 0.50% (w/w) phenoxyethanol; about 0.20% (w/w) 3-(trimethoxysilyl)propyldimethyl octadecyl ammonium chloride and about 99.2% (w/w) deionized water |
| Neutralization Method | Dilution |
| Product Diluent | Distilled water |
| Test Concentration | Neat (80%), Mid-range, Non active |
| Experimental Conditions | Clean |
| Interfering Substance | Clean 0.3g/l Bovine Albumin (grams per liter) |
| Test Temperature | 20°C +/- 1°C |
| Temperature of Incubation | 37°C +/- 1°C |
| Identification of the Viral Strains: | Modified vaccinia virus Ankara (MVA), ATCC VR-1508 |
| Contact Times | 5 minutes + 10s (seconds) |
| Stability and Appearance During Test | No Change Observed (Homogenous) |

The acceptance criteria for the disinfectant composition when tested according to BS EN 14476 is that the disinfectant composition when tested as above shall demonstrate at least a 4 log₁₀ reduction against the test virus. The test is deemed valid where all control requirements are met. The tested hand sanitizer received has achieved a 4-log reduction against Vaccinia virus when tested under the condition stipulated above.

### Example 9

### (Viral Biocidal and Longevity Activity Hand Sanitizer)

Example 9 is a study which evaluated the performance, characteristics, efficacy and usability to meet the needs of large numbers of people for a SARS-CoV-2 of a hand sanitizer comprised of about 0.10% (w/w) benzalkonium chloride; about 0.50% (w/w) phenoxyethanol; about 0.20% (w/w) 3-(tri-methoxysilyl)propyldimethyl octadecyl ammonium chloride and about 99.2% (w/w) deionized water. Three experiments were run (a positive control, a test and a negative control) where a total of 50 pairs of gloves were used. In the first experiment -a positive control-, twenty pairs of gloves were soiled with SARS COV 2 confirmed specimens and then swabbed and tested for SARS COV 2 at 1hr (hour), 6 hours and 24 hours post exposure.

In the second experiment, twenty pairs of gloves were first treated using the hand sanitizer, let to dry and then contaminated with the same SARS COV 2 positive specimens. They were also swabbed and tested for SARS COV 2 at 1 hour, 6 hours and 24 hours post exposure. In a third experiment, as a negative control, 10 pairs of gloves were exposed to double distilled water, let to dry, and tested 1, 6 and 24 hours post exposure.

All swabs were tested using the Abbott Realtime Polymerase Chain reaction for SARS COV 2. Two health care professionals were involved in the study and filled a questionnaire on usability. For the first experiment -a positive control-, the swabs taken from all the 20 pairs of gloves that were contaminated with SARS COV 2 specimens tested coronavirus positive at the 1, 6 and 24 hour intervals. In the second experiment, swabs taken from all the 20 pairs of gloves that were first sanitized with the hand sanitizer and then contaminated with SARS COV 2 specimens tested coronavirus negative at both the 1, 6 and 24 hour intervals. In the third experiment -negative control-, 10 pairs of gloves that were exposed to double distilled water, tested negative at 1, 6 and 24 hours post exposure.

In the foregoing, the efficacy of the hand sanitizer for SARS-CoV-2 was 100% at 1 and 6 hours. The efficacy was 100% at 1, 6 and 24 hours.

Although the present invention has been described in considerable detail with reference to certain preferred versions thereof, other versions are possible with substituted, varied and/or modified materials and steps are employed. These other versions do not depart from the invention. Therefore, the spirit and scope of the appended claims should not be limited to the description of the preferred versions contained herein.

The invention also provides the following aspects:
[1] A kill and protect surface disinfectant composition comprised of:
   a. between about 0.20% (w/w) to about 1.00% (w/w) benzalkonium chloride;
   b. between about 0.25% (w/w) to about 3.00% (w/w) hydrogen peroxide;
   c. between about 0.25% (w/w) to about 0.75% (w/w) 3-(trimethoxysilyl)propyldimethyl octadecyl ammonium chloride and
   d. between about 95.25% (w/w) to about 99.30% deionized water, such that the weight percentage total of all the ingredients is 100%.
[2] The kill and protect surface disinfectant composition of [1] where:
   a. the benzalkonium chloride is that of the same which meets the description of Chemical Abstracts Service No. 68424-85-1 and
   b. the hydrogen peroxide is that of the same which meets the description of Chemical Abstracts Service No.7722-84-1 and
   c. the 3-(tri-methoxysilyl)propyldimethyl octadecyl ammonium chloride is that of the same which meets the description of Chemical Abstracts Service No. 27668-52-6;
[3] A surface sanitation method comprised of the steps of:
   a. applying to a surface by spraying, wiping or fogging the kill and protect surface disinfectant composition of [1] or [2];
   b. allowing the benzalkonium chloride and hydrogen peroxide to kill:
      i. microorganisms which are present on the surface and
      ii. microorganisms which are transferred to the surface by landing aerial particles or droplets or contacting bodies, at about the time of the applying;
   c. allowing the composition to dry on the surface with the covalent bonding of the 3-(tri-methoxysilyl)propyldimethyl octadecyl ammonium chloride to form a positively charged micro thin coating on the surface which electrostatically clings negatively charged microorganisms, pierces their outer membrane or coat and kills the microorganism,
   whereby for a period of at least about 30 days there is a killing of microorganisms which are transferred to the surface by landing aerial particles or droplets or contacting bodies.
[4] A kill and protect surface disinfectant composition comprised of:
   a. about 0.50% (w/w) benzalkonium chloride;
   b. about 0.50% (w/w) hydrogen peroxide;
   c. about 0.50% (w/w) 3-(tri-methoxysilyl)propyldimethyl octadecyl ammonium chloride and
   d. about 98.5% (w/w) deionized water.
[5] The kill and protect surface disinfectant composition of [4] where:
   a. the benzalkonium chloride is that of the same which meets the description of Chemical Abstracts Service No. 68424-85-1;
   b. the hydrogen peroxide is that of the same which meets the description of 7722-84-1 and
   c. the 3-(tri-methoxysilyl)propyldimethyl octadecyl ammonium chloride is that of the same which meets the description of Chemical Abstracts Service No. 27668-52-6.
[6] A surface sanitation method comprised of the steps of:
   a. applying to a surface by spraying, wiping or fogging the kill and protect surface disinfectant composition of [4] or [5];
   b. allowing the benzalkonium chloride and hydrogen peroxide to kill:
      i. microorganisms which are present on the surface and
      ii. microorganisms which are transferred to the surface by landing aerial particles or droplets or contacting bodies, at about the time of the applying;
   c. allowing the composition to dry on the surface with the covalent bonding of the 3-(tri-methoxysilyl)propyldimethyl octadecyl ammonium chloride to form a positively charged micro thin coating on the surface which electrostatically clings negatively charged microorganisms, pierces their outer membrane or coat and kills the microorganism,
   whereby for a period of at least about 30 days there is a killing of microorganisms which are transferred to the surface by landing aerial particles or droplets or contacting bodies.
[7] A hand sanitizer composition comprised of:
   a. between about 0.05% (w/w) to about 0.20% (w/w) benzalkonium chloride;
   b. between about 0.20% (w/w) to about 0.50% (w/w) 3-(trimethoxysilyl)propyldimethyl octadecyl ammonium chloride;
   c. between about 0.25% (w/w) to about 0.75% (w/w) phenoxyethanol and
   d. between about 98.55% (w/w) to about 99.5% (w/w) deionized water, such that the weight percentage total of all the ingredients is 100%.
[8] The hand sanitizer composition of [6] where the:
   a. the benzalkonium chloride is that of the same which meets the description of Chemical Abstracts Service No. 68424-85-1;
   b. the 3-(tri-methoxysilyl)propyldimethyl octadecyl ammonium chloride is that of the same which meets the description of Chemical Abstracts Service No. 27668-52-6
   c. the phenoxyethanol is that of the same which meets the description of Chemical Abstracts Service No. 122-99-6 and
   d. the deionized water is that of the same which meets the description of Chemical Abstracts Service No. 7732-18-5.
[9] The hand sanitizer composition of [7] having between about 0.05% (w/w) to about 0.15% (w/w) fragrance with there being proportionally less deionized water such that the weight percentage total of all the ingredients is 100%.
[10] The hand sanitizer composition of [9] where the fragrance is selected from the group consisting of bergamot, gardenia, honeysuckle, jasmine, lavender, pear, peony, rosemary, sandalwood and vanilla.
[11] A hand sanitation method comprised of the steps of:
   a. applying generously to hands a hand sanitizer composition of [7], [8], [9] or [10];
   b. rubbing hands together so as to spread the hand sanitizer thoroughly about the hands and
   c. allowing the hand sanitizer to remain in a liquid state on the hands for at least about a minute,
   whereby for a period of at least about 24 hours there is a killing of microorganisms.
[12] A hand sanitizer composition comprised of:
   a. about 0.10% (w/w) benzalkonium chloride;
   b. about 0.20% (w/w) 3-(tri-methoxysilyl)propyldimethyl octadecyl ammonium chloride;
   c. about 0.50% (w/w) phenoxyethanol and
   d. about 99.2% (w/w) deionized water.
[13] The hand sanitizer composition of [12] where the:
   a. the benzalkonium chloride is that of the same which meets the description of Chemical Abstracts Service No. 68424-85-1;
   b. the 3-(tri-methoxysilyl)propyldimethyl octadecyl ammonium chloride is that of the same which meets the description of Chemical Abstracts Service No. 27668-52-6
   c. the phenoxyethanol is that of the same which meets the description of Chemical Abstracts Service No. 122-99-6 and
   d. the deionized water is that of the same which meets the description of Chemical Abstracts Service No. 7732-18-5.
[14] The hand sanitizer composition of [12] having about 0.1% (w/w) fragrance with there being about 99.1 (w/w) deionized water such that the weight percentage total of all the ingredients is 100%.
[15] The hand sanitizer composition of [14] where the fragrance is selected from the group consisting of bergamot, gardenia, honeysuckle, jasmine, lavender, pear, peony, rosemary, sandalwood and vanilla.
[16] A hand sanitation method comprised of the steps of:
   a. applying generously to hands a hand sanitizer composition of [12], [13], [14] or [15];
   b. rubbing hands together so as to spread the hand sanitizer thoroughly about the hands and
   c. allowing the hand sanitizer to remain in a liquid state on the hands for at least about a minute,
   whereby for a period of at least about 24 hours there is a killing of microorganisms.
[17] A method of providing a community benefit towards controlling the spread of infection where the community is comprised of humans and surfaces all to which humans interact comprised of the steps of:
   a. the sanitizing of surfaces by applying the kill and protect surface disinfectant composition of [1], [2], [3], [4] or [5] to surfaces and
   b. the sanitizing of the hands of humans by applying the hand sanitizer composition of [7], [8], [9], [10], [12], [13], [14] or [15] to hands of humans.

## Claims

1. A composition comprised of:
a. between about 0.05% (w/w) to about 1.00% (w/w) benzalkonium chloride;
b. between about 0.25% (w/w) to about 3.00% (w/w) of a component selected from hydrogen peroxide and/or phenoxyethanol;
c. between about 0.20% (w/w) to about 0.75% (w/w) of 3-(trimethoxysilyl)propyldimethyl octadecyl ammonium chloride;
d. up to about 0.15% (w/w) fragrance;
e. about 95.10% (w/w) to about 99.50% (w/w) deionized water,
such that the weight percentage total of all the ingredients is 100%.

2. A kill and protect surface disinfectant composition comprised of:
a. between about 0.05% (w/w) to about 1.00% (w/w) benzalkonium chloride;
b. between about 0.25% (w/w) to about 3.00% (w/w) hydrogen peroxide;
c. between about 0.25% (w/w) to about 0.75% (w/w) 3-(trimethoxysilyl)propyldimethyl octadecyl ammonium chloride and
d. between about 95.25% (w/w) to about 99.45% deionized water,
such that the weight percentage total of all the ingredients is 100%.

3. A kill and protect surface disinfectant composition comprised of:
a. about 0.50% (w/w) benzalkonium chloride;
b. about 0.50% (w/w) hydrogen peroxide;
c. about 0.50% (w/w) 3-(tri-methoxysilyl)propyldimethyl octadecyl ammonium chloride and
d. about 98.5% (w/w) deionized water.

4. The kill and protect surface disinfectant composition of claims 2 or 3 where:
a. the benzalkonium chloride is that of the same which meets the description of Chemical Abstracts Service No. 68424-85-1;
b. the hydrogen peroxide is that of the same which meets the description of 7722-84-1 and
c. the 3-(tri-methoxysilyl)propyldimethyl octadecyl ammonium chloride is that of the same which meets the description of Chemical Abstracts Service No. 27668-52-6.

5. A surface sanitation method comprised of the steps of:
a. applying to a surface by spraying, wiping or fogging the kill and protect surface disinfectant composition of any one of claims 2 to 4;
b. allowing the benzalkonium chloride and hydrogen peroxide to kill:
i. microorganisms which are present on the surface and
ii. microorganisms which are transferred to the surface by landing aerial particles or droplets or contacting bodies, at about the time of the applying;
c. allowing the composition to dry on the surface with the covalent bonding of the 3-(tri-methoxysilyl)propyldimethyl octadecyl ammonium chloride to form a positively charged micro thin coating on the surface which electrostatically clings negatively charged microorganisms, pierces their outer membrane or coat and kills the microorganism,
whereby for a period of at least about 30 days there is a killing of microorganisms which are transferred to the surface by landing aerial particles or droplets or contacting bodies.

6. A hand sanitizer composition comprised of:
a. between about 0.05% (w/w) to about 0.20% (w/w) benzalkonium chloride;
b. between about 0.20% (w/w) to about 0.50% (w/w) 3-(trimethoxysilyl)propyldimethyl octadecyl ammonium chloride;
c. between about 0.25% (w/w) to about 0.75% (w/w) phenoxyethanol and
d. between about 98.55% (w/w) to about 99.5% (w/w) deionized water, such that the weight percentage total of all the ingredients is 100%.

7. The hand sanitizer composition of claim 6 having between about 0.05% (w/w) to about 0.15% (w/w) fragrance with there being proportionally less deionized water such that the weight percentage total of all the ingredients is 100%.

8. The hand sanitizer composition of claim 7 where the fragrance is selected from the group consisting of bergamot, gardenia, honeysuckle, jasmine, lavender, pear, peony, rosemary, sandalwood and vanilla.

9. A hand sanitizer composition comprised of:
a. about 0.10% (w/w) benzalkonium chloride;
b. about 0.20% (w/w) 3-(tri-methoxysilyl)propyldimethyl octadecyl ammonium chloride;
c. about 0.50% (w/w) phenoxyethanol and
d. about 99.2% (w/w) deionized water.
e.

10. The hand sanitizer composition of claim 9 having about 0.1% (w/w) fragrance with there being about 99.1 (w/w) deionized water such that the weight percentage total of all the ingredients is 100%.

11. The hand sanitizer composition of claim 10 where the fragrance is selected from the group consisting of bergamot, gardenia, honeysuckle, jasmine, lavender, pear, peony, rosemary, sandalwood and vanilla.

12. The hand sanitizer composition of any one of claims 6 to 11 where the:
a. the benzalkonium chloride is that of the same which meets the description of Chemical Abstracts Service No. 68424-85-1;
b. the 3-(tri-methoxysilyl)propyldimethyl octadecyl ammonium chloride is that of the same which meets the description of Chemical Abstracts Service No. 27668-52-6
c. the phenoxyethanol is that of the same which meets the description of Chemical Abstracts Service No. 122-99-6 and
d. the deionized water is that of the same which meets the description of Chemical Abstracts Service No. 7732-18-5.

13. A hand sanitation method comprised of the steps of:
a. applying generously to hands a hand sanitizer composition of any one of claims 6 to 12;
b. rubbing hands together so as to spread the hand sanitizer thoroughly about the hands and
c. allowing the hand sanitizer to remain in a liquid state on the hands for at least about a minute,
whereby for a period of at least about 24 hours there is a killing of microorganisms.

14. A method of providing a community benefit towards controlling the spread of infection where the community is comprised of humans and surfaces all to which humans interact comprised of the steps of:
a. the sanitizing of surfaces by applying the kill and protect surface disinfectant composition of any one of claims 2 to 4 to surfaces and
b. the sanitizing of the hands of humans by applying the hand sanitizer composition of any one of claims 6 to 12 to hands of humans.
